# EUROPEAN PATENT APPLICATION

(11) **EP 3 238 681 A1**
(43) Date of publication of application: **01.11.2017**
(21) Application number: 17171663.2
(22) Date of filing: 25.05.2016
(51) Int. Cl.: A61F 13/84, G06Q 30/06

(54) **A METHOD OF MANUFACTURING AND MARKETING CUSTOMIZED ABSORBENT HYGIENE ARTICLES**

(30) Priority: 28.05.2015 EP 15169520
(62) Divisional of application: 16171207.0
(71) Applicant: The Procter & Gamble Company, Cincinnati, OH 45202 (US)
(72) Inventor: SCHMIDT, Mattias, 65824 Schwalbach (DE); FEIST, Barry Robert, Cincinnati, OH 45202 (US); GREENING II, Nelson Edward, Cincinnati, OH 45202 (US); ISELE, Olaf Erik Alexander, Cincinnati, OH 45202 (US); KANYA, Kevin Ronald, Cincinnati, OH 45202 (US); LANGE, Stephen Joseph, Cincinnati, OH 45202 (US); RAYCHECK, Jeromy Thomas, Cincinnati, OH 45202 (US); ROE, Donald Carroll, Cincinnati, OH 45202 (US)
(74) Representative: Mather, Peter Geoffrey

(57) **Abstract**

The present invention relates to a method of manufacturing customized absorbent hygiene articles, the absorbent hygiene articles comprising an absorbent core (7), a topsheet (8), and a backsheet (9), the method comprising the steps of:
a) providing a set of choices to allow a consumer to specify the customized absorbent hygiene article;
b) receiving a selection from the consumer among the set of choices;
c) manufacturing the customized absorbent hygiene article; and
d) distributing the customized absorbent hygiene article to the consumer;
wherein the set of choices is selected from the group consisting of:
i) the material specification of the backsheet (9);
ii) the material specification and/or design of leg cuffs (25);
iii) the material specification and/or design of waist bands or waist elastics;
iv) the material specification and/or design of fastening members (22) or elastic side panels;
v) the material specification and/or design of a raised barrier for fecal control; and combinations thereof.

## Description

### FIELD OF THE INVENTION

The present invention relates to a method of manufacturing and marketing customized absorbent hygiene articles.

### BACKGROUND OF THE INVENTION

In recent years, in various industries, mass customization has come to be seen as an alternative or as a complement to mass production. The most well-known examples of mass customization include the business model developed by Dell® in the market for personal computers, and Nike-ID for providing customized shoes. Also, in the market for cars, especially at the higher end of the market, manufacturers provide the opportunity for purchasers of new cars to select from a range of options, including both technical, such as engine type and size, in-car entertainment options etc., and aesthetic, such as colour of exterior, colour, fabric and style of interior seats and trim etc. Each car ordered is then made to order on modem production lines.

Attempts at applying mass customization methods to fast moving consumer goods markets, such as food, clothing and other household items, have met with mixed commercial success.

Various services offer customization by printing text and/or graphics, onto packaging. Services also offer customization by printing text and/or graphics such as a baby's name on to already manufactured diapers. These examples of personalization are generally focused upon aesthetic appearance by means of printing onto the outer package, or onto the outer cover of a diaper.

WO2013/144728, published on October 3rd 2013, discloses method and apparatus for producing and marketing tailored on-demand disposable garments and undergarments for hygiene products.

In "Cracking the code of mass customization", MIT Sloane Management Review, 2009, the authors F. Salvador, P. Martin de Holan and F. Piller report studies into over 200 manufacturing operations. They adopt a definition attributed to Joseph Pine that the term mass customization means: "developing, producing, marketing and delivering affordable goods and services with enough variety and customization that nearly everyone finds exactly what they want." The authors suggest that successful mass customization requires a business to develop three fundamental capabilities: 1) the ability to identify the product attributes along which customer needs diverge, 2) the ability to reuse or recombine existing organizational and value-chain resources, and 3) the ability to help customers identify or build solutions to their own needs.

There remains a need for providing methods of mass customization in other fast moving consumer goods industries. In particular, there remains a need for providing methods of mass customization and personalization for hygiene articles that offer choices beyond simple aesthetic printing options.

### SUMMARY OF THE INVENTION

The present invention relates to a method of manufacturing customized absorbent hygiene articles, the absorbent hygiene articles comprising an absorbent core, a topsheet, a backsheet, the method comprising the steps of:
a) providing a set of choices designed to allow a consumer to specify the customized absorbent hygiene article;
b) receiving a selection from the consumer among the set of choices;
c) manufacturing the customized absorbent hygiene article; and
d) distributing the customized absorbent hygiene article to the consumer;
wherein the set of choices is selected from the group consisting of:
the material specification of the topsheet and/or the backsheet;
the material specification and/or design of leg cuffs;
and combinations thereof.

In another embodiment of the invention the set of choices is selected from the group consisting of the absorbent capacity, absorbent profile, and/or shape of the absorbent core.

In another embodiment of the invention the set of choices is selected from the group consisting of:
the material specification and/or design of waist bands or waist elastics;
the material specification and/or design of fastening members or elastic side panels;
the material specification and/or design of a raised barrier for fecal control; and combinations thereof.

### BRIEF DESCRIPTION OF THE DRAWINGS

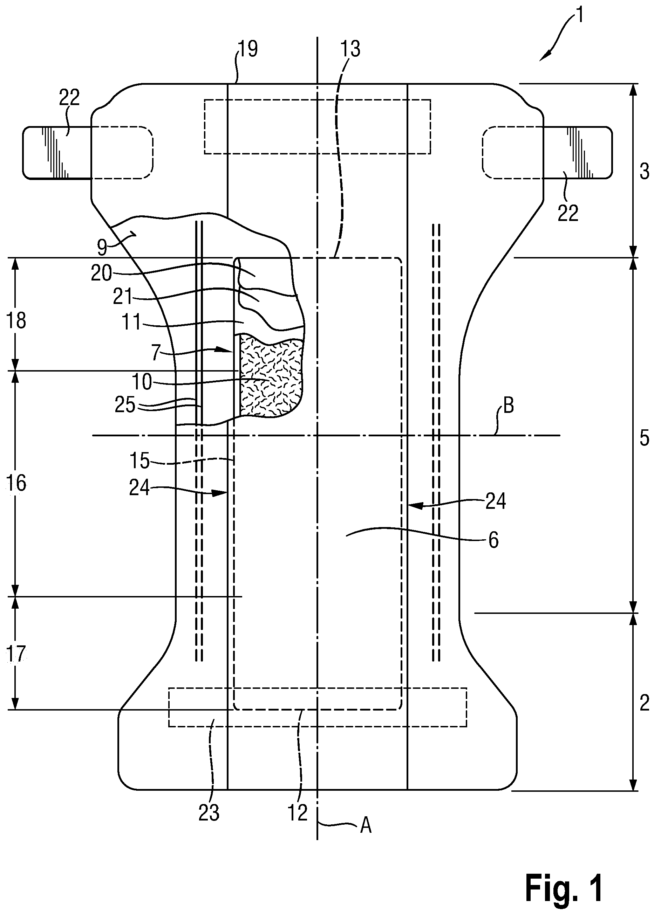

Figure 1 is a plan view of an absorbent hygiene article.

### DETAILED DESCRIPTION OF THE INVENTION

When absorbent hygiene articles are designed and manufactured for the mass market, the product specifications are selected in order to meet the needs of the vast majority of consumers. However, in reality, all consumers are different both in terms of their needs and in terms of their desired preferences.

Mass customization, therefore, aims to provide specific products which cater to the needs of each specific consumer. The present invention provides a method for manufacturing and marketing which presents the consumer with enhanced ability to identify and build solutions to their own needs.

Each consumer selects from a set of choices, preferably by navigating through one or more pages which may be presented to the consumer by a web-based interface. The web-based interface may be provided using a personal or lap-top computer, or a mobile device such as a mobile telephone, smartphone or tablet. In some cases the consumer may directly specify their preferences (for example, with or without lotion and/or fragrance added to the article), whilst in other cases the consumer may record a preference which is translated by the manufacturer, preferably by the manufacturer's software, into a design specification (for example, the consumer may indicate high, medium or low absorbent capacity, which will be translated into a technical specification for the absorbent core adapted for that consumer).

Preferably the interface is a web-based interface comprising various navigation tools. A web-based interface offers the advantages of enabling consumers to be informed or advised about certain product attributes. The on-line interface may also satisfy a consumer's need for discretion when seeking information, advice, or making a purchase.

The consumer may be presented by a simple list of choices from which to select their preferences (i.e. a "menu"), and/or the consumer may be presented with one or more "packages" which help to direct their preferences. For example, packages may be designated as "protection package", "skin care package", "luxury package", and so on.

The general construction of a topsheet, suitable for use in a baby or adult incontinence article or in a feminine hygiene article, will be described in more detail below. According to the present invention the topsheet may be tailored towards each consumer's specific needs or wishes is a number of ways. The material specification may be tailored to the consumer's specific needs. The consumer may prefer materials which have a particular desired softness or texture, for example which have different bulk or thickness, which have different bonding patterns etc.

The general construction of elastic members, such as leg cuffs, leg elastics, barrier cuffs will be described in more detail below. According to one embodiment the topsheet and/or leg cuff may have lotion applied thereon. Consumers may select different lotion types, for example skin care lotion and/or lotion loading, or alternatively, some consumers may prefer no lotion at all.

The general construction of an absorbent core will be described in more detail below. According to the present invention the absorbent core may be tailored towards each consumer's specific needs or wishes is a number of ways. The absorbent capacity of the absorbent core may be varied according to each consumers needs. Moreover the absorbent core may have a variable absorbent profile and/or be a shaped core (i.e. non-rectangular). According to one embodiment the profile of the absorbent core may be varied in such a way that the absorbent capacity per unit area is greater in one unit area than in another unit area. This may enable customization of the absorbent core for absorbent hygiene articles for females and for males, for example.

Optionally the set of choices from which each consumer may select may further include aesthetic items, such as graphics and/or fragrances, and may further include packaging specifications. Packaging specifications may include different types of package, different pack count (i.e. the number of absorbent articles per pack), decoration and graphics applied to the package.

The term "customized patterns" as used herein includes printed patterns, embossed patterns, textured surface and/or aperture sheet. Printed patterns include decoration and graphics which may be selected by the consumer from a gallery of pictures, designs and text offered by the manufacturer via the interface, and/or decoration and graphics may be uploaded via the interface by the consumer who may wish to have their own images, text represented on the absorbent article.

The term "absorbent hygiene articles" as used herein refers to diapers, training pants, adult incontinence products, feminine hygiene products, etc. Preferably, absorbent hygiene articles are disposable. "Disposable" refers to devices which are generally not intended to be laundered or otherwise restored or reused (i.e., they are intended to be discarded after a single use and, may be recycled, composted or otherwise disposed of in an environmentally compatible manner). The absorbent hygiene article may be completely disposable, or may be partly disposable. For example, the absorbent hygiene article could comprise a customized disposable absorbent core which could be associated in use with a reusable (i.e. non-disposable) product chassis.

The term "leg cuffs" as used herein comprises leg gasketing systems, leg elastics and barrier cuffs.

Referring now to Figure 1, absorbent hygiene articles in the context of the present invention comprise a liquid pervious topsheet **8** that faces the wearer's body, a liquid impervious backsheet **9** that faces the wearer's clothing and an absorbent core **7** interposed between the liquid pervious topsheet **8** and the backsheet **9.**

The liquid pervious topsheet **8** is preferably a nonwoven material comprising fibers. "Nonwoven material" as used herein refers to a manufactured web of directionally or randomly orientated fibers, excluding paper and products which are woven, knitted, tufted, stitch-bonded incorporating binding yarns or filaments, or felted by wet-milling, whether or not additionally needled. Nonwoven materials and processes for making them are known in the art. Generally, processes for making nonwoven materials comprise laying fibers onto a forming surface, which may comprise spunlaying, meltblowing, carding, airlaying, wetlaying, coform and combinations thereof. The fibers may be of natural or man-made origin and may be staple fibers or continuous filaments or be formed in situ.

Typically, bonding between the fibers of the nonwoven material imparts strength to the material. Strength is needed to provide resistance to tearing during use of the absorbent hygiene article and also during the manufacturing process. Softness, or perception of softness, is also a desirable property of the topsheet. Softness may be increased by, for example, decreasing the bonding area, but consequently strength may be adversely affected.

The topsheet may be a nonwoven material made of synthetic fibers alone or in combination with natural fibers. Examples of natural fibers may include cotton, cellulosic natural fibers, such as fibers from hardwood sources, softwood sources, starches or other non-wood plants. The synthetic fibers can be any material, such as, but not limited to, those selected from the group consisting of polyolefins (polypropylene and polypropylene copolymers, polyethylene and polyethylene copolymers), polyesters (e.g., polyethylene terephthalate), polyethers, polyamides, polyesteramides, polyvinylalcohols, polyhydroxyalkanoates, polysaccharides, and combinations thereof. Further, the synthetic fibers can be a single component (i.e. a single synthetic material or a mixture makes up the entire fiber), bi-component (i.e. the fiber is divided into regions, the regions including two or more different synthetic materials or mixtures thereof and may include coextruded fibers and core and sheath fibers) and combinations thereof. Bi-component fibers can be used as a component fiber of the nonwoven material, and/or they may be present to act as a binder for the other fibers present in the nonwoven material. Any or all of the fibers may be treated before, during, or after manufacture to change any desired properties of the fibers. Preferably, the topsheet comprises at least 20% of synthetic fibers, or at least 30% of synthetic fibers or at least 50% of synthetic fibers. In some embodiments, the topsheet comprises 100% of synthetic fibers. Synthetic fibers are preferably thermoplastic fibers. Preferably, the topsheet is made of a nonwoven material made of a polyolefin, such as polyethylene, polypropylene or mixtures thereof. The topsheet may be a multilayer nonwoven web, i.e. a laminate. The laminate may comprise spunbond layer(s) (S), and/or meltblown layer(s) (M), and/or carded layer(s) (C). Suitable laminates include, but are not limited to, SS, SSS, SMS or SMMS. In some embodiments, the topsheet is a spunbond nonwoven material, such as a mono layer spunbond (S), or a dual layer spunbond (SS) or a nonwoven material comprising more than two layers, such as a spunbond nonwoven with three layers (SSS).

The topsheet **8** and/or the leg cuff **25** may, optionally, comprise an applied lotion. For example the lotion may comprise a skin care ingredient or a skin enhancing ingredient. A lotion may also provide anti-stick properties to reduce the adhesion of fecal material to the topsheet **8**.

The backsheet **9** may be vapor pervious but liquid impervious. The backsheet may prevent the fluids absorbed and contained in the absorbent structure from wetting materials that contact the absorbent article such as underpants, pants, pyjamas, undergarments, and shirts or jackets, thereby acting as a barrier to fluid transport. The backsheet can also allow the transfer of at least water vapor, or both water vapor and air through it. The backsheet may be elastic or inelastic. In some embodiments the backsheet may be a nonwoven fabric. In some embodiments, the backsheet may comprise a laminate of a nonwoven and a thin plastic film such as a thermoplastic film having a thickness of 0.012 mm to 0.051 mm. Suitable backsheet films include those manufactured by Tredegar Industries Inc. of Terre Haute, Ind. and sold under the trade names X15306, X10962, and X10964; or those manufactured by Clopay.

The absorbent hygiene article 1 further comprises an absorbent core **7** disposed between the topsheet **8** and the backsheet **9**. The absorbent core **7** typically comprises absorbent material **10** such as cellulose fibers, modified cellulose fibers (cellulose fibers and modified cellulose fibers are typically referred to in the art as "air-felt"), particulate absorbent polymer material, absorbent foams, tissue, or mixtures thereof. "Particulate absorbent polymer material" as used herein refers to substantially water-insoluble polymer particles that can absorb at least 5 times their weight of a 0.9% saline solution in de-ionized water as measured using the Centrifuge Retention Capacity test (Edana 441.2-01). Suitable particulate absorbent polymer material can be selected among polyacrylates and polyacrylate based materials, such as for example partially neutralized cross-linked polyacrylates or acid polyacrylate. Examples of polyacrylate based polymers very slightly cross-linked, or substantially not crosslinked at all are described in the PCT Patent Application WO 07/047598.

In some embodiments, the absorbent core **7** may comprise less than 5% by weight of cellulose, more typically less than 2% and most typically is cellulose free. The resulting absorbent structures have a reduced thickness in the dry state compared to conventional absorbent structure comprising cellulosic fibers. The reduced thickness helps to improve the fit and comfort of the absorbent article for the wearer.

In some embodiments, the absorbent core **7** may comprise one or more absorbent structures, each absorbent structure comprising a nonwoven substrate layer supporting particulate absorbent polymer material, said particulate absorbent polymer material being immobilized on the nonwoven substrate layer by a thermoplastic adhesive composition, which preferably forms a fibrous network over the particulate absorbent polymer material. Suitable thermoplastic adhesive compositions includes hot melt adhesives comprising at least a thermoplastic polymer in combination with a plasticizer and other thermoplastic diluents such as tackifying resins and additives such as antioxidants. Exemplary suitable hot melt adhesive materials are described in EP 1447067 A2. In some embodiments, the thermoplastic polymer has a molecular weight of more than 10,000 and a glass transition temperature usually below room temperature or comprised from -6 °C to 16°C. In some embodiments, the concentration of the thermoplastic polymer in a hot melt is in the range of about 20 to about 40% by weight. The thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof. The thermoplastic adhesive composition is generally present in the form of fibres forming a fibrous network over the particulate absorbent polymer material. The fibres may have an average thickness from about 1 µm to about 100 µm, or from about 25 µm to about 75 µm, and an average length from about 5 mm to about 50 cm. The thermoplastic adhesive composition may be applied at an amount of from 0.5 to 30 g/m², or from 1 to 15 g/m², or from 1 and 10 g/m² or even from 1.5 and 5 g/m² per substrate layer. In some embodiments, the particulate absorbent polymer material is distributed in clusters of particles on the nonwoven substrate layer. In some embodiments, the absorbent structure comprises less than 5%, or less than 2% by weight of cellulose, or is cellulose free. The nonwoven substrate layer may enclose the absorbent polymer material and the thermoplastic composition or a separate nonwoven substrate layer may cover the absorbent polymer material and the thermoplastic composition. These nonwoven substrate layers are therefore often referred to as core wrap or core cover. The core wrap or core cover may consist of a first, upper nonwoven web **11** towards the body-facing surface of the absorbent article and of a second, lower nonwoven web (not shown in the figure) towards the garment-facing surface of the absorbent article. The first and second nonwoven webs may be continuously or intermittently bonded to each other around their perimeters. The first and second substrate layers may be made of the same nonwoven webs or may be made of different nonwoven webs, i.e. the first, upper substrate layer may be fluid pervious whereas the second, lower substrate layer may be fluid impervious. In a multilayer absorbent core, one or more layers of a substrate (e.g. a nonwoven web) may additionally be placed within the absorbent core to at least partially separate and segment the particulate absorbent polymer material. The core wrap or core cover may be present in any types of absorbent core.

The periphery of the absorbent core 7 is defined by the outer absorbent core edges wherein the absorbent core front waist edge **12** and back waist edges **13** run between the longitudinal edges **15** generally parallel to the transverse axis **A** of the absorbent hygiene article. The longitudinal dimension of the absorbent core **7** extends along the longitudinal axis **A** from the absorbent core front waist edge **12** to the absorbent core back waist edge **13**. The absorbent core **7** also has a transverse dimension extending along the transverse axis **B**, which is running perpendicular to the longitudinal axis **A**. The absorbent core **7** has an absorbent core crotch region **16**, an absorbent core front waist region **17** which extends from the absorbent core crotch region **16** to the absorbent core front waist edge **12.** The absorbent core back waist region **18** extends from the absorbent core crotch region **16** to the absorbent core back waist edge **13.** The absorbent core has a crotch region **16**.

The configuration and construction of the absorbent core **7** may comprise an absorbent profile, e.g. the absorbent core **7** may have varying caliper zones, a hydrophilic gradient, a superabsorbent gradient, or lower average density and lower average basis weight acquisition zones. Further, the size and absorbent capacity of the absorbent core may be varied to accommodate wearers ranging from babies and infants through adults. Customization of the capacity of the absorbent core may be particularly advantageous for adult incontinence products for which the range of degree of incontinence, and therefore the absorbent capacity need, may vary widely.

In some embodiments, the absorbent material **10** (e.g. the particulate absorbent polymer material) may be distributed unevenly along the longitudinal dimension of the absorbent core **7**. For example, the absorbent core crotch region **16** may comprise a higher amount of absorbent material **10** per area compared to the absorbent core front and back waist regions **17** and **18**. In some embodiment, the absorbent core back waist region **18** comprises not more than 0.01 gram of absorbent material per cm² surface area (i.e. from 0 g of absorbent material per cm² to 0.01 gram per cm²). In those embodiments, the absorbent core front waist region **17** preferably comprises at least 0.03 gram of absorbent material per cm², more preferably at least 0.04 gram per cm². The absorbent core crotch region **16** preferably comprises at least 0.04 gram of absorbent material per cm², more preferably at least 0.05 gram per cm². The amount of absorbent material in the respective region is calculated by determining the weight of absorbent material in this region and is dividing it by the total surface area of the region (hence, the average amount is taken).

In some embodiment, every area of the absorbent core back waist region **18** comprises not more than 0.01 gram of absorbent material per cm². Also, every area of the absorbent core front waist region **17** may comprise at least 0.03 gram of absorbent material per cm², preferably at least 0.04 grams per cm², and every area of the absorbent core crotch region **16** may comprise at least 0.04 gram of absorbent material per cm², preferably at least 0.05 gram per cm². In a preferred embodiment, the absorbent material **10** comprises at least 90 weight % of particulate absorbent polymer material, more preferably at least 95 weight % and even more preferably 100 weight %.

In some embodiments, such as shown in Figure 1, the absorbent core back waist edge **13** is longitudinally offset by at least 10% from the chassis back waist edge **19**, as it has been found that the absorbent core **7** is not use of in that area of the absorbent hygiene article. The 10% are determined based on the longitudinal extension of the chassis **6**. Preferably, the absorbent core back waist edge **13** is longitudinally offset by at least 12% from the chassis back waist edge **19**, more preferably by at least 15%. These configurations result in a distribution of the absorbent material only in these regions of the absorbent hygiene article where absorption of liquid is really needed and thus contribute to reduce the bulk of the absorbent hygiene article. The amount of absorbent material may also vary within the absorbent core, e.g. the absorbent core may be profiled in its longitudinal direction to provide most of the absorbent capacity in the front half of the absorbent hygiene article where the liquid discharge occurs predominately. Despite the reduced bulk, it has been found that the removal of the absorbent material in the back waist region of the absorbent hygiene article may be not always easily accepted by some consumers. The absorbent hygiene article may further comprise an acquisition system disposed between the topsheet **8** and a wearer facing side of the absorbent core **7**, for instance between the cover layer **11** of the absorbent core 7 and the topsheet **8**. The acquisition system may be in direct contact with the absorbent core 7. The acquisition system may comprise a single layer or multiple layers, such as an upper acquisition layer **20** facing towards the wearer's skin and a lower acquisition layer **21** facing the garment of the wearer. The acquisition system may function to receive a surge of liquid, such as a gush of urine. In other words, the acquisition system may serve as a temporary reservoir for liquid until the absorbent core can absorb the liquid. The acquisition layer can for example comprise fibrous nonwoven materials made by air laying or wet laying of synthetic fibres such as polyethylene (PE), polyethylene terephthalate (PET), or polypropylene (PP), similarly to the cover layer of the absorbent core. Exemplary materials for the fluid acquisition layer could comprise spunbonded or carded nonwoven materials, or air laid materials such as for example latex bonded or thermal bonded airlaid materials. Basis weights can typically range from about 10 g/m² to about 60 g/m², or from about 25 g/m² to about 40 g/m².

In order to keep the absorbent hygiene article 1 in place about the wearer, at least a portion of the chassis back waist region **3** may be attached by the fastening members **22** to at least a portion of the chassis front waist region **2** to form leg opening(s) and an article waist. According to certain embodiments, the disposable absorbent hygiene article may be provided with a re-closable fastening system joined to the chassis for securing the absorbent hygiene article to a wearer, or may alternatively be provided in the form of a pant-type disposable diaper. The fastening system may include at least one fastening member **22** and at least one landing zone **23**. In a pant-type disposable diaper, the article may comprise side panels joined to the backsheet **9** and/or topsheet **8** along their longitudinal edges facing towards the longitudinal axis **A** and joined to each other along their longitudinal edges facing away from the longitudinal axis **A** to form a pant. Side panels are preferably stretchable for ease of putting on and fitting the absorbent hygiene article around the waist, and for comfort during wearing.

The absorbent hygiene article **1** may further comprise elastic members such as leg cuffs. The term "leg cuffs" as used herein comprises leg gasketing systems, leg elastics **25** and barrier cuffs. Suitable leg cuffs are described hereinafter, and also in for example U.S. 3,860,003; U.S. 4,808,178; U.S. 4,695,278 and 4,795,454.

Suitable processes for assembling the absorbent hygiene article are preferably high speed mass production processes. By high speed it is to be understood that modem mass production of absorbent hygiene articles typically runs at at least 600 articles per minute, preferably at least 800 articles per minute, more preferably at least 1000 articles per minute, and most preferably at least 1200 articles per minute. For economic reasons, most or all of the absorbent hygiene articles are manufactured at such high speeds, however it is not excluded that some part of the customization might be carried out using slower process operations.

Processes for assembling the absorbent hygiene article include conventional techniques known in the art for constructing and configuring disposable absorbent articles such as adhesive, ultrasonic bonding, thermal point bonding via conduction or convection, high pressure roatary bonding or radiofrequency point bonding as described in, for example, U.S. 4,854,984, incorporated herein by reference. For example, the backsheet and/or the topsheet can be joined to the absorbent core or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or adhesive manufactured by Bostik, of Wauwatosa, Wisconsin under the designation H-2031.

In one embodiment of the invention the absorbent core may, as noted above, vary in its configuration and construction, e.g. the absorbent core may have varying caliper zones, a hydrophilic gradient, a superabsorbent gradient, or lower average density and lower average basis weight acquisition zones.

Such variations are referred to herein as the "absorbent profile" of the absorbent core. The absorbent core may be profiled in the machine direction and/or in the cross machine direction. Profiling may include regions in which absorbent material is substantially or completely absent. Such regions may be in the form of longitudinal or shaped lines, such as channels. Other such regions may take the form of "cut-outs", for example shaped areas around the edge of the absorbent core which are substantially or completely free of absorbent material.

Improved liquid transportation can be achieved by the provision of transportation channels for distributing liquid in the absorbent article, e.g., the absorbent structure thereof. Furthermore, it has been found that improved fit can be obtained by providing absorbent articles with absorbent structures wherein the absorbent material is structured in longitudinal direction, optionally with regions that comprise less or no absorbent material, for improved bending flexibility in use.

Absorbent articles comprising regions of the absorbent core which are substantially or completely free of absorbent material, and processes for making such articles, are described, for example, in WO2012/170778, and WO2012/170798, both published on December 13th 2012, incorporated herein by reference.

The overall shape of the finished absorbent article is defined by the layered structure comprising the topsheet, the backsheet, and the absorbent core, as described above. Together with additional elements which make up the finished article, for example, leg elastics, leg cuffs, waist elastics, waist band, the overall shape of the absorbent article is referred to as the "chassis".

According to particular aspects of the present invention the chassis may comprise leg cuffs, shaped profile, optionally with opacity patch, and/or waistbands, as described below.

### Leg Cuff:

According to a particular aspect of the invention the leg gasketing system comprises both an inner cuff and an outer cuff. The inner cuff of the leg gasketing system comprises an inner cuff folded edge and an inner cuff material edge. The outer cuff comprises an outer cuff folded edge and an outer cuff material edge. The cuff may be made of a single web of material such that the web of material is folded laterally inward to form the outer cuff folded edge and folded laterally outward to form the inner cuff material edge. The leg gasketing system may extend from the first waist edge to the second waist edge may be joined to the topsheet and/or backsheet, or other layers in between the inner cuff folded edge and the outer cuff folded edge in the crotch region. The folded web may be an N-fiber material, which therefore allows the folded outer leg cuff to not comprise a polymeric film.

The leg cuff provides a folded outer leg cuff design having finished edges with elastics that are close to the edge to maintain a close proximity to the skin to create improved fit, a more aesthetically pleasing, clothing-like design and improved leakage protection. It is also desirable that the article have a folded outer leg cuff design that does not have a polymeric film in the elasticized region and still prevents penetration of exudates and molten adhesive.

### Shaped Chassis/Strengthening Patch:

The chassis may be profiled (e.g. wider at the waist regions, narrower in the crotch) to provide a more garment like appearance. Because the outer cuff may be made with N-fiber material and therefore does not need a polymeric film, the polymeric film is narrower and does not extend all the way to the cuffs. Because of the narrower crotch width of the shaped chassis, the polymeric film is narrower than the narrowest crotch width. For extra support in the waist regions, an extra layer of material is used to provide increased support for when the ear tab is pull in applying the diaper. The extra layer of material may be an elastic patch or an opacity strengthening patch which also provides increased opacity for a more garment like appearance.

### Waistbands:

Absorbent articles can also include a front waistband and/or a back waistband attached to the waist regions of the chassis. The waistbands can have consolidation of the gathers, which means the waistband operates so that when the waistband is extended to its maximum stretching capacity, there are still gathers of consolidated material present in the waistband. This provides a cushion between the user and the diaper, even when the waistband is fully stretched, providing a more garment like feel. The waistbands can also have differentiation between the front and back of the diaper. This means that the front waistband may be designed to have more or less stretch than the back waistband, providing a more snug fit.

### Zones:

The absorbent hygiene articles may comprise two or more zones, such as three zones, four zones, five zones etc. The zones may be positioned at any suitable locations on a wearer-facing surface, on a garment facing surface, or both, of an absorbent hygiene article. A first zone may surround, or at least partially surround, a second zone. One zone may be positioned in a laterally central strip of the wearer-facing surface and another zone may form two laterally outboard strips on the wearer-facing surface on both sides of the laterally central strip. A first zone may be positioned on a first side of a lateral axis of the absorbent article and a second zone may be positioned on a second side of the lateral axis of the absorbent article. One zone may be positioned on a first side of a generally laterally-extending transverse barrier and another zone may be positioned on a second side of the generally laterally-extending transverse barrier.

The zones may each comprise one or more features. The features may include three-dimensional structures, three-dimensional structures with apertures, two layer three-dimensional structures where portions of one layer extend through portions of the other layer, two layer three-dimensional structures where portions of one layer are nested together with portions of another layer, apertures, patterned apertures, embossments, fusion bonds, inks, dyes, flow control materials, surfactants, lotions, and/or waxes, for example. The features in the zones can be tailored to specific purposes of the zones, such as urine management, fecal management, menses management, and/or other purposes.

### Raised barrier:

The absorbent hygiene articles may comprise a wearer-facing surface that includes a topsheet. A raised barrier may extend upwardly (i.e., out of the diaper in a z direction) relative to a portion of the topsheet. The raised barrier may be positioned on the topsheet or positioned under the topsheet. The raised barrier may at least partially surround, or fully surround, at least a portion of, or all of, an absorbent core. The raised barrier may be positioned inboard of the absorbent core, outboard of the absorbent core, or over at least a portion of the absorbent core. One purpose of the raised barrier is to retain one or more bodily fluids over a portion of the absorbent core (and at least inhibit outward x and y directional wicking) so that the one or more bodily fluids can be efficiently absorbed into the absorbent core. The raised barrier may be made of any suitable materials, such as nonwoven materials, foams, waxes, and/or plastics, for example.

In one embodiment of the invention the raised barrier is a transverse barrier. The transverse barrier is extensible in order to accommodate the presence of fecal material deposited in the rear portion of the diaper. When the transverse barrier is extended, the volume of the fecal material is accommodated without breaching the transverse barrier and allowing smearing of fecal material in the front portion of the diaper onto the wearer's skin.

The longitudinal and lateral axes define the plane of the absorbent article. The absorbent article has a Z-direction orthogonal to the plane of the diaper and orthogonal to the plane defined by the longitudinal and lateral axes of the absorbent article. The absorbent article further has a periphery defined by a front waist margin, a rear waist margin, and two longitudinal side margins connecting the front and rear waist margins.

The absorbent article further comprises a liquid pervious topsheet, a liquid impervious backsheet joined to the topsheet, an absorbent core intermediate the topsheet and the backsheet, and a pair of upstanding barrier leg cuffs. Each barrier leg cuff extends outwardly from a proximal end joined to the topsheet to a distal end spaced from the proximal end. The barrier leg cuffs are generally longitudinally oriented.

The absorbent article has a fecal material containing volume. The fecal material containing volume is defined by and contained within the proximal and distal ends of the transverse barrier and the barrier leg cuffs. The fecal material containing volume is increasable upon the deposition of fecal material therein and which intercepts the transverse barrier.

The transverse barrier may be extensible, whereby the fecal material containing volume is increasable upon the receipt of fecal material therein. Furthermore, the transverse barrier may be elastically extensible. The transverse barrier may comprise an elastic panel. The panel may be elastically extensible in the longitudinal direction, the lateral direction, or bilaterally extensible.

Alternatively, the elasticity may be provided by a series of elastic strands. In yet another embodiment, the extensibility need not be elastic, but may, for example, be provided by pleats having releasable frangible bonds. The bonds are broken upon receipt of or pressure due to the presence of fecal material. Any such embodiment is sufficient, so long as the fecal material containing volume is increasable upon receipt of fecal material therein, and preferably which does increase in response to the presence of fecal material contacting the transverse barrier.

Absorbent articles comprising transverse barriers are described, for example, in WO98/56327, published on December 17^{th} 1998, incorporated herein by reference.

Conventionally absorbent hygiene articles are sold in the mass market in a limited number of sizes, for example five or six different sizes of diapers to cover the development from a new born baby up to about a 4 or 5 year old child. However mass customisation offers the opportunity to supply the customers with any desired size, including sizes which would be intermediate, within the range of currently marketed products. Customisation in terms of size would be particularly desirable in the market for adult incontinence products because adult humans vary considerably in size and shape (much more so than human babies/infants).

Mass customisation also offers the opportunity to customise the shape and proportions of the chassis of the absorbent hygiene article, and/or to customise the shape and proportions of the constituent parts of the article, for better fit and functionality. In order to achieve this the consumer may enter one or more key physical dimensions of the body via the interface. An example of a key physical dimension is waist size. In addition, or alternatively, the consumer may provide size information via the interface using a scan or photograph in a form which can be interpreted into size information by software.

Mass customisation also offers the opportunity to combine an assortment of absorbent hygiene articles into one pack (i.e. one shipping unit) to a consumer. For example a consumer may prefer to receive a certain proportion of absorbent articles designed for use during the day, together with the remaining portion of absorbent articles designed for use during the night. A consumer may prefer a proportion of absorbent articles designed and packaged for use at home, together with a proportion of absorbent articles designed and packaged for use on-the-go, i.e. outside of the home. Some consumers may prefer different assortments of graphics etc.

The number of absorbent hygiene articles in each pack (i.e. each shipping unit) may be customized in order to meet the consumer's wishes.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method of manufacturing customized absorbent hygiene articles, the absorbent hygiene articles comprising an absorbent core (7), a topsheet (8), and a backsheet (9), the method comprising the steps of:
a) providing a set of choices to allow a consumer to specify the customized absorbent hygiene article;
b) receiving a selection from the consumer among the set of choices;
c) manufacturing the customized absorbent hygiene article; and
d) distributing the customized absorbent hygiene article to the consumer;
**characterized in that** the set of choices is selected from the group consisting of:
i) the material specification of the backsheet (9);
ii) the material specification and/or design of leg cuffs (25);
iii) the material specification and/or design of waist bands or waist elastics;
iv) the material specification and/or design of fastening members (22) or elastic side panels;
v) the material specification and/or design of a raised barrier for fecal control; and
vi) combinations thereof.

2. A method of manufacturing according to Claim 1 wherein the set of choices further comprises the selection of a lotion applied to the leg cuffs (25).

3. A method of manufacturing according to Claim 2 wherein the lotion applied to the leg cuffs (25) comprises a skin enhancing ingredient.

4. The method according to any of Claims 1 to 3 wherein the set of choices further comprises selection and/or intensity of a fragrance applied to the absorbent hygiene article.

5. The method according to any of Claims 1 to 4 wherein the set of choices further comprises consumer customized patterns which are: selected by the consumer from a gallery; or uploaded by the consumer; or combination thereof.

6. The method of any of Claims 1 to 5 wherein the set of choices further comprises an assortment of different absorbent hygiene articles in a shipping unit.

7. The method of Claim 6 wherein the assortment of different absorbent articles comprises an assortment of different absorbent hygiene articles wherein the different absorbent hygiene articles have different customized patterns.

8. The method of any of Claims 1 to 5 wherein the set of choices further comprises the number of absorbent hygiene articles in a shipping unit.

9. The method of any of claims 1 to 8 wherein at least one of steps a) or b) is carried out using a web-based interface.

10. The method of any of claims 1 to 9 wherein each one of steps a) and b) is carried out using a web-based interface.
